(19) Europäisches Patentamt
European Patent Office
Office européen des brevets

(11) Numéro de publication: **0 275 855 B1**

(12) **FASCICULE DE BREVET EUROPEEN**

(45) Date de publication du fascicule du brevet: **22.11.90**

(51) Int. Cl.[5]: **C07C 15/04**, C07C 7/13

(21) Numéro de dépôt: **87870174.7**

(22) Date de dépôt: **10.12.87**

(54) **Procédé de purification du benzène.**

(30) Priorité: **21.01.87 FR 8700647**

(43) Date de publication de la demande: **27.07.88 Bulletin 88/30**

(45) Mention de la délivrance du brevet: **22.11.90 Bulletin 90/47**

(84) Etats contractants désignés: **AT BE CH DE ES FR GB GR IT LI LU NL SE**

(56) Documents cités:
**CHEMICAL ABSTRACTS, vol. 101, no. 1, 2 juillet 1984, page 577, résumé no. 6790h, Columbus, Ohio, US; & SU-A-1 065 392 (R.SH. KHARITONOVA et al.) 07-01-1984**

(73) Titulaire: **N.V. SOPAR S.A., Rue Ducale, 29, B-1000 Bruxelles(BE)**

(72) Inventeur: **Mingels, Walter, 32 Burg Chalmetlaan, B-9060 Zelzate(BE)**
Inventeur: **Enödy, Eva Marra, 138 Prins Boudewijnlaan, B-2610 Wilrijk(BE)**
Inventeur: **Vansant, Etienne Frans Maria, 6 Manderleylaan, B-2553 Zoersel(BE)**

(74) Mandataire: **Grisar, Daniel et al, c/o Office Kirkpatrick Sprl. 4, square de Meeûs, B-1040 Bruxelles(BE)**

**EP 0 275 855 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé de purification de benzène obtenu par la distillation des benzols bruts.

Les benzols bruts contiennent essentiellement du benzène, du toluène, des xylènes et des impuretés, comme du cyclopentadiène, du dicyclopentadiène, du cyclohexane, du méthylcyclohexane, des hydrocarbures aliphatiques comme l'heptane et le diméthylbutane et, en outre, du thiophène qui est très difficile à séparer du benzène.

Le procédé suivant l'invention peut s'appliquer à la purification de benzols bruts de diverses origines et principalement aux benzols bruts de cokerie.

Le procédé de traitement général des benzols bruts consiste en une distillation primaire qui permet principalement de séparer du benzène le toluène et les xylènes et d'autres fractions, un traitement pour séparer le thiophène du benzène impur et une nouvelle distillation pour éliminer d'autres impuretés et de l'eau, pour obtenir un benzène pur.

Dans tous les procédés de traitement des benzols bruts, il peut être nécessaire d'éliminer ou de séparer le thiophène du benzène. Les procédés classiques utilisés à cet effet industriellement sont le raffinage à l'acide sulfurique concentré et l'hydrogénation.

Le premier procédé permet d'amener la teneur en thiophène à environ 10 ppm. L'addition dans certaines conditions de composés insaturés permet d'atteindre une teneur en thiophène de 2 ppm. Ce système présente toutefois des désavantages, comme la manipulation d'un acide agressif avec les problèmes de corrosion et les dangers que cela représente, des pertes de produits par sulfonation partielle et la production d'un acide résiduaire dont l'élimination est généralement difficile et coûteuse.

Le second procédé permet une élimination totale du thiophène. Toutefois, cette technique présente des inconvénients, comme la nécessité de travailler avec de l'hydrogène à des pressions et températures élevées et la formation de produits non aromatiques qui constituent également des impuretés difficiles à éliminer par la suite.

Il y a donc un intérêt évident à trouver une méthode plus efficace et moins coûteuse pour la séparation du thiophène du benzène.

L'invention a dès lors pour but de traiter du benzène impur par une méthode plus efficace que les méthodes actuellement connues. De manière plus particulière, l'invention a pour but de traiter du benzène impur par une méthode qui ne nécessite pas la manipulation de produits corrosifs et dangereux, tels que l'acide sulfurique, et qui évite au maximum les problèmes que pose l'évacuation d'acides résiduaires ou autres substances polluantes. L'invention a également pour but de traiter du benzène impur par une méthode qui permet de séparer le benzène de grande pureté à un taux de récupération très élevé, sans qu'il soit nécessaire de travailler avec de l'hydrogène à des pressions et températures élevées.

Or, la Demanderesse a trouvé de manière surprenante que ces divers buts peuvent être atteints en effectuant le traitement du benzène impur par une méthode qui fait usage d'un certain type de zéolite synthétique modifiée.

L'emploi de tamis moléculaires pour séparer des mélanges de composés aromatiques a déjà été décrit dans l'art antérieur. Par exemple, il est connu par les brevets américains nº 3 668 267, 3 772 399, 3 840 610 et 4 014 949 que par un tamis moléculaire consistant en une zéolite cristalline qui a été partiellement déshydratée, on peut obtenir une adsorption sélective d'un hydrocarbure cyclique d'un mélange avec un autre hydrocarbure cyclique ayant une structure similaire.

Il est également connu par le brevet américain nº 4 188 285 que par mise en contact de l'essence avec une zéolite du type faujasite échangée avec des ions d'argent, on peut sélectivement enlever le thiophène de l'essence.

Toutefois, l'état de la technique ne révèle aucune description d'un procédé de séparation du thiophène du benzène faisant usage d'une zéolite.

La présente invention concerne un procédé pour l'épuration du benzène impur contenant du thiophène et d'autres impuretés. Le procédé de l'invention comprend :

- la mise en contact de benzène impur à épurer avec une zéolite d'alumino-silicate cristalline Y qui a subi un échange d'ions avec une solution aqueuse d'un composé de cuivre bivalent, un séchage et un traitement thermique progressif de conditionnement jusqu'à une température comprise entre 200 et 550°C, et
- la régénération de la zéolite chargée de thiophène et d'autres impuretés, comprenant un traitement thermique progressif de régénération jusqu'à une température comprise entre 200 et 550°C pour préparer la zéolite à une nouvelle mise en contact avec du benzène impur à épurer.

La solution aqueuse du composé de cuivre bivalent est, par exemple, une solution de chlorure de cuivre.

Après que l'échange d'ions soit complet, on lave la zéolite à l'eau distillée et ensuite on la sèche à l'air pendant au moins 12 heures à environ 50°C.

Le traitement thermique de conditionnement de la zéolite se fait ensuite dans une colonne, en faisant passer dans celle-ci un courant d'air sec dont on augmente la température à une allure ne dépassant pas 5°C par minute jusqu'à environ 140°C. On maintient la température à 140°C jusqu'à ce que l'air sortant de la colonne ne contienne plus d'humidité, puis on augmente de nouveau la température à une allure ne dépassant pas 10°C par minute jusqu'à une température comprise entre 200 et 550°C, on maintient le courant d'air à cette température au moins jusqu'à ce qu'il y ait uniformité de température dans la colonne et on refroidit finalement la colonne par passage d'un courant d'air sec non chauffé.

Avantageusement, le traitement thermique de conditionnement se fait jusqu'à une température comprise entre 300 et 400°C, par exemple jusqu'à environ 350°C.

Suivant une forme d'exécution, le benzène impur à épurer est mis en contact avec la zéolite à l'état liquide, à une température comprise entre 20 et 80°C à pression atmosphérique ou bien sous pression et à une température supérieure à 80°C.

Suivant une autre forme d'exécution, le benzène impur à épurer est mis en contact avec la zéolite à l'état de vapeur.

La mise en contact du benzène impur à épurer avec la zéolite peut se faire en discontinu, avantageusement en ajoutant la zéolite au benzène impur et en le maintenant un certain temps sous agitation.

Cependant, la mise en contact du benzène impur à épurer avec la zéolite se fait de préférence en continu. Dans ce cas, on fait de préférence passer le benzène impur dans une colonne chargée de la dite zéolite.

Pour la régénération de la zéolite, on chasse d'abord les liquides et les vapeurs de la colonne avec un courant d'azote dont on augmente la température à une allure ne dépassant pas 10°C par minute jusqu'à environ 150°C, on remplace le courant d'azote par un courant d'air sec dont on continue à augmenter la température à une allure ne dépassant pas 10°C par minute jusqu'à une température comprise entre 200 et 550°C, on maintient le courant d'air à cette température, au moins jusqu'à ce qu'il y ait uniformité de température dans la colonne, et on refroidit finalement la colonne jusqu'à la température d'utilisation de la zéolite, par passage d'un courant d'air sec non chauffé.

Avantageusement, le traitement thermique de régénération de la zéolite se fait jusqu'à une température comprise entre 300 et 400°C, par exemple jusqu'à environ 350°C.

La zéolite d'alumino-silicate cristalline modifiée utilisée dans le procédé suivant l'invention est la zéolite du type Y qui a subi un échange d'ions avec du cuivre bivalent; elle sera appelée Cu(II)Y, dans la suite de la description.

L'invention est illustrée ci-après par des exemples, et également en se référant aux dessins annexés, dans lesquels :

la Fig. 1 est un diagramme de percée du benzène, du thiophène et des hydrocarbures divers d'une colonne chargée de Cu(II)Y, lors de sa première utilisation pour l'épuration du benzène impur;

la Fig. 2 est un diagramme de percée analogue pour la même colonne après régénération de la zéolite, et

la Fig. 3 est un diagramme correspondant aux résultats de l'essai décrit à l'exemple 6 donné ci-après.

<u>Préparation de la zéolite Cu(II)Y.</u>

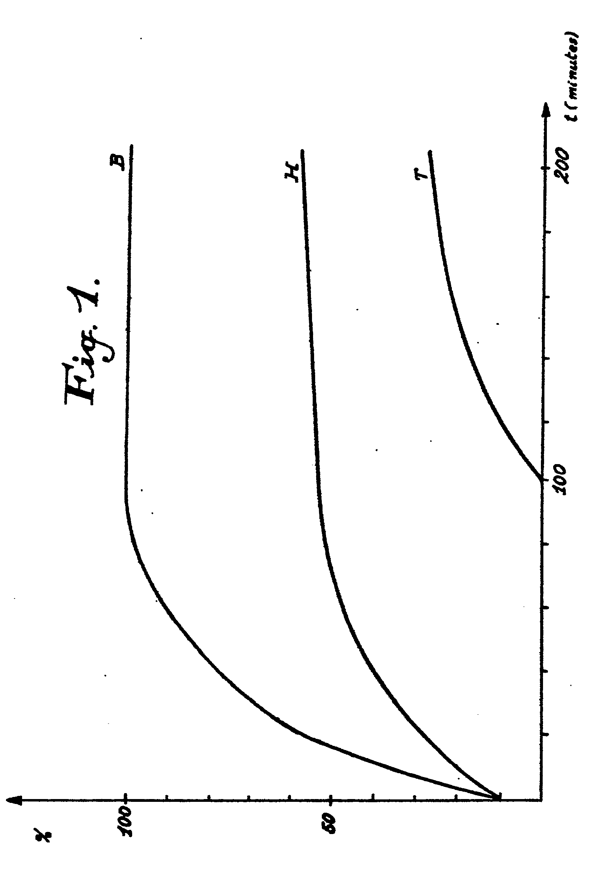

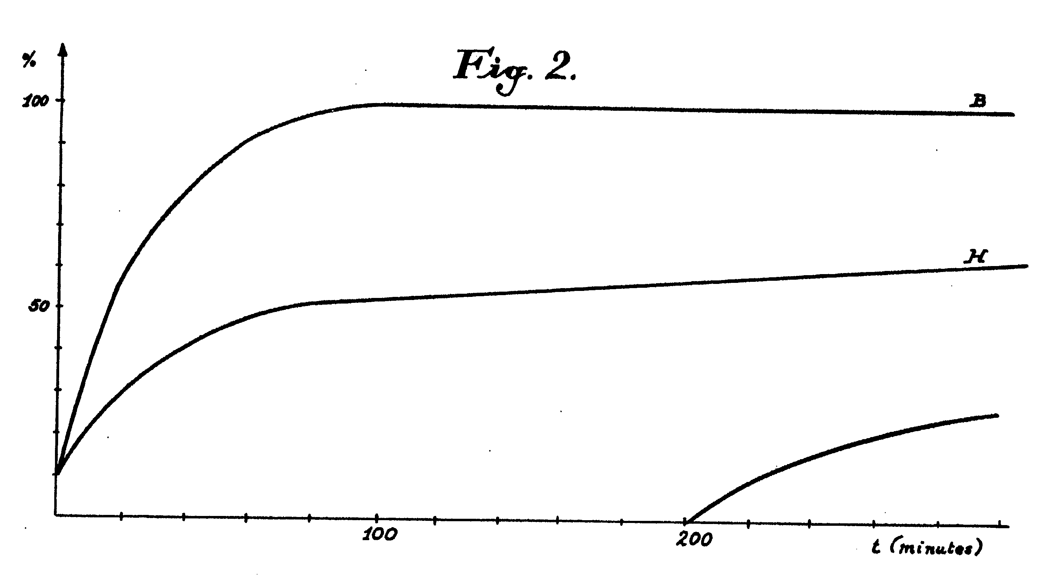

Dans les essais illustrés par les Fig. 1 et 2 et dans les exemples qui suivent, il est fait usage de la zéolite Cu(II)Y dont les particules ont une dimension moyenne allant de 0,150 à 0,250 mm (60 à 100 mesh) agglomérées en granules d'une dimension moyenne de 1,6 mm (1/16 pouce).

La capacité d'échange de la zéolite sodique du type Y est de 4,34 milliéquivalents par gramme.

La forme cuivrique de cette zéolite est préparée par échange d'ions avec une solution d'un sel de cuivre bivalent, telle qu'une solution aqueuse de chlorure de cuivre.

Pour obtenir un échange complet, il importe de mettre la zéolite en contact avec une solution de $CuCl_2$ contenant une quantité nettement supérieure à la quantité stoechiométrique (par exemple cinq fois supérieure). La zéolite est maintenue en contact avec la solution pendant au moins 2 heures. Pendant ce temps, le mélange est maintenu à environ 40-50°C et est agité régulièrement. La zéolite est alors séparée de la solution par filtration, lavée à l'eau distillée et ensuite séchée à l'air dans une étuve ventilée, à environ 50°C, pendant au moins 12 heures.

Il est à noter que l'échange d'ions qui constitue la première étape de la préparation de la zéolite Cu(II)Y peut également être réalisé en chargeant la zéolite dans une colonne et en faisant traverser le lit de la zéolite par une solution de $CuCl_2$, par exemple à 40-50°C, jusqu'à ce que l'échange d'ions soit complet.

Après le séchage à température peu élevée, la zéolite est soumise à un traitement thermique de conditionnement. Pour subir ce traitement thermique de conditionnement qui est une étape très importante du procédé, la zéolite est de préférence placée dans une colonne pouvant être chauffée et traversée par un courant d'air sec à température réglable. La colonne elle-même est isolée et, si nécessaire, chauffée extérieurement.

Au début d'une première phase du traitement thermique de conditionnement, l'air est amené à la colonne à une température de 50°C en maintenant un débit d'air (ou d'un gaz contenant de l'oxygène) suffisant pour assurer un bon échange de chaleur avec la zéolite. La température de l'air amené dans la colonne est alors augmentée progressivement à raison de maximum 5°C par minute.

Le débit d'air et l'allure de la montée en température doivent être adaptés à la taille et la géométrie de la colonne : il importe, en effet, que l'augmentation de température ne soit pas trop rapide et que la température soit pratiquement uniforme dans tout le lit de zéolite.

Lorsqu'on a atteint la température de 140°C, on maintient cette température aussi longtemps que l'air sortant de la colonne révèle la présence d'humidité.

On a, en effet, pu observer qu'il importe de ne pas dépasser la température de 150°C tant que l'air sortant de la colonne contient de l'humidité, sans quoi le cuivre bivalent de la zéolite est réduit en cuivre monovalent et que sous cette forme, la zéolite est inefficace pour l'épuration du benzène.

Lorsque l'air sortant de la colonne ne révèle plus la présence d'humidité, on augmente de nouveau la température à raison de 10°C par minute jusqu'à 350°C et on maintient cette température et le courant d'air pendant au moins 1 heure, mais en tout cas jusqu'à ce qu'il y ait uniformité de température dans la colonne. Le chauffage est alors arrêté, mais le courant d'air sec et non chauffé est maintenu dans la colonne jusqu'au moment où la zéolite soit refroidie jusqu'à la température d'utilisation prévue.

Epuration du benzène.

Le benzène impur à épurer est amené à passer au travers de la colonne contenant la zéolite Cu(II)Y avec un débit adapté à la taille et la géométrie de la colonne, de manière à assurer un temps de contact suffisant pour réduire la teneur en thiophène du benzène ortant au niveau souhaité.

Régénération de la zéolite Cu(II)Y.

Pour la régénération de la zéolite, le liquide et/ou les vapeurs présents dans la colonne sont chassés au moyen d'un courant d'azote.

La température est portée progressivement à 150°C à raison de 10°C par minute. Quand la température de 150°C est atteinte, on remplace le courant d'azote par un courant d'air sec et on augmente de nouveau la température à raison de 10°C par minute jusqu'à 350°C et on maintient cette température et le courant d'air jusqu'à ce qu'il y ait uniformité de température dans la colonne.

Le réglage de cette augmentation de température est obtenu comme pour le traitement thermique effectué lors du premier conditionnement de la zéolite.

Le chauffage est alors arrêté, mais le courant d'air sec et non chauffé est maintenu dans la colonne jusqu'à ce que la zéolite soit refroidie jusqu'à la température d'utilisation prévue.

Les diagrammes montrés aux Fig. 1 et 2 concernent des essais de laboratoire effectués avec une colonne en verre d'une hauteur de 10 cm et d'un diamètre de 3,5 mm, remplie de Cu(II)Y dans laquelle on fait passer, avec un gaz porteur (azote), du benzène impur obtenu par une première distillation fractionnée d'un benzol brut, et contenant 99% de benzène pur, 0,5% de thiophène et 0,5% d'autres impuretés qui sont essentiellement des hydrocarbures. Dans les conditions opératoires de cet essai, 90% en volume du courant gazeux passant par la colonne est de l'azote et le débit de ce mélange gazeux est de 15 ml par minute.

Ces diagrammes montrés aux Fig. 1 et 2 indiquent, en fonction du temps, les teneurs en benzène (B), en thiophène (T) et en impuretés hydrocarbonées diverses (H), dans le mélange gazeux sortant de la colonne, par rapport à la teneur de chacun de ces constituants dans le mélange gazeux entrant dans la colonne.

En prolongeant la durée de l'essai, on constate évidemment, qu'après un certain temps, les trois courbes (B, T et H) atteindraient le niveau 100%, indiquant la saturation de la zéolite (équilibre entre la teneur en B, T et H dans la zéolite et dans le mélange entrant dans la colonne).

Le diagramme de la Fig. 1 montre que cette colonne a un temps de percée très bref pour le benzène, et un temps de rétention du thiophène de 110 minutes. Ce diagramme montre également que la colonne retient environ 45% des autres impuretés, pendant la durée de l'essai qui est arrêté après 180 minutes.

La Fig. 2 qui est un diagramme de percée de cette même colonne de zéolite après une sixième régénération de celle-ci, montre que lors du septième cycle de fonctionnement, la colonne a un temps de rétention du thiophène de 200 minutes. Pendant la durée du septième cycle de fonctionnement qui est interrompu après 240 minutes, la colonne retient environ 55% des autres impuretés.

On a pu constater que l'efficacité de fonctionnement de la colonne de zéolite est augmentée après la première régénération. Elle est légèrement supérieure à l'efficacité lors du premier cycle de fonctionnement et se maintient statistiquement au même niveau.

L'invention est également illustrée ci-après par des exemples non limitatifs.

EXEMPLE 1 - (Préparation de la zéolite Cu(II)Y).

La zéolite du type Y, d'une dimension moyenne de 0,150 à 0,250 mm, est chargée dans une colonne et on fait traverser ce lit de zéolite par une solution de $CuCl_2$ à 45°C. Ensuite, la zéolite est lavée à l'eau distillée jusqu'à l'absence de chlorure dans l'eau de lavage.

La zéolite ainsi traitée est séchée à l'air dans une étuve ventilée à environ 50°C pendant 12 heures. On place la zéolite dans une colonne de 50 cm de long et de 5 cm de diamètre. Au départ, on assure à travers la zéolite un débit d'air chaud suffisant pour assurer un bon échange de température.

La colonne est chauffée à raison de maximum 5°C par minute. Lorsqu'on obtient 140°C, on maintient cette température jusqu'à ce qu'on ne puisse plus détecter de l'humidité dans l'air sortant de la colonne.

Ensuite, on augmente la température de nouveau à raison de 10°C par minute jusqu'à 350°C et on maintient cette température et le courant d'air jusqu'à ce qu'il y ait homogénéité de température dans la colonne. Ensuite, on arrête le chauffage, mais on maintient la zéolite sous courant d'air en la refroidissant jusqu'à la température d'utilisation prévue.

EXEMPLE 2.-

A 33 ml de benzène impur, on ajoute 10 g de la zéolite traitée, comme décrit dans l'exemple 1, et on les maintient à 25°C sous agitation. On prélève des échantillons régulièrement et on mesure la teneur en thiophène.

Les résultats suivants en % de thiophène dans le benzène par rapport à la teneur initiale, sont obtenus :

| Temps (minutes) | Thiophène, % |
|---|---|
| 0 | 100 |
| 10 | 60 |
| 20 | 40 |
| 30 | 30 |
| 40 | 25 |
| 50 | 22 |

EXEMPLE 3.-

L'exemple 2 est répété à 60°C. Les résultats suivants sont obtenus :

| Temps (minutes) | Thiophène, % |
|---|---|
| 0 | 100 |
| 10 | 15 |
| 20 | 5 |
| 30 | 2 |
| 50 | 2 |
| 70 | 1,5 |

On a pu constater que l'efficacité de la zéolite pour la séparation du thiophène augmente avec l'augmentation de la température.

EXEMPLE 4.-

Une colonne de 50 cm de haut et d'un diamètre de 4 cm est chargée de 500 ml de zéolite Y traitée comme décrit dans l'exemple 1. On y fait passer en circuit fermé 675 g de benzène contenant initialement 3470 ppm de thiophène. La température du contenu de la colonne est maintenue entre 50 et 60°C. Le débit de circulation est maintenu à 7 litres par heure. On constate une diminution régulière de la teneur en thiophène dans le benzène. Cette teneur se stabilise à 850 ppm après 2 heures de fonctionnement.

EXEMPLE 5.-

L'exemple 4 est répété en faisant passer du benzène ontenant initialement 73,6 ppm de thiophène. Après 90 minutes de fonctionnement, la teneur en thiophène dans le benzène est inférieure à 0,1 ppm.

EXEMPLE 6.-

On place en série deux colonnes de 50 cm de haut et d'un diamètre de 4 cm, contenant chacune 500 ml de zéolite Y traitée comme décrit dans l'exemple 1, et on alimente du benzène contenant 73,6 ppm de thiophène à un débit de 0,5 litre par heure.

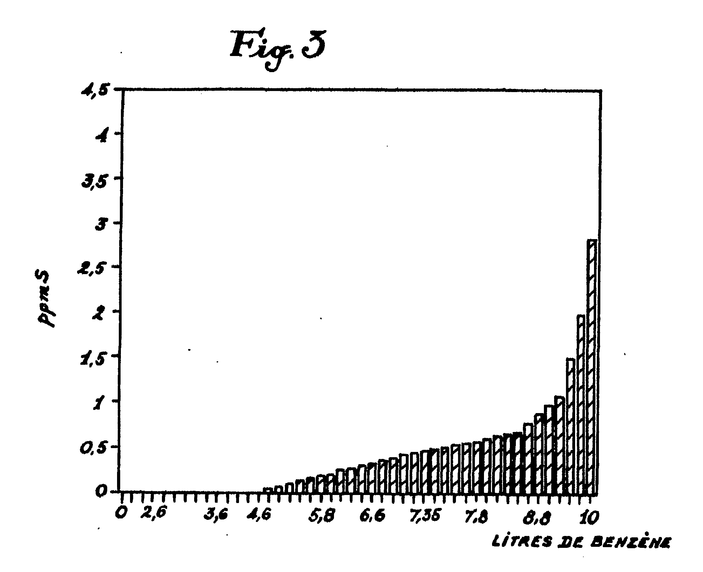

La Fig. 3, qui est un diagramme de percée de ces deux colonnes en série, montre que pour les dix premières heures de fonctionnement, soit pour les cinq premiers litres de benzène, il n'y a pratiquement pas de percée pour le thiophène. Avec la saturation progressive de la zéolite, il y a une augmentation correspondante de la teneur en thiophène exprimée sur le diagramme en ppm de soufre).

Après traitement de 10 litres de benzène, soit 20 heures de fonctionnement, la teneur moyenne en thiophène du benzène sortant de la première colonne est de 10,2 ppm (3,9 ppm de soufre) et la teneur moyenne en thiophène du benzène sortant de la seconde colonne est de 1,3 ppm, ce qui correspond à moins de 0,5 ppm de soufre.

EXEMPLE 7.-

Une colonne de 75 cm de haut et d'un diamètre de 5 cm est chargée de 725 g de zéolite Y traitée comme décrit dans l'exemple 1, et on alimente du benzène contenant 4320 ppm de thiophène à un débit de 0,5 litre par heure.

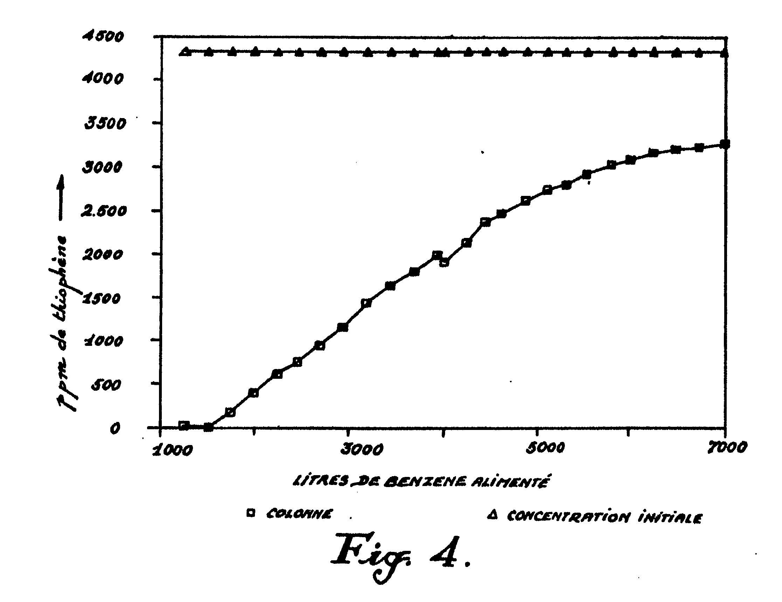

La Fig. 4 montre l'évolution de la teneur en thiophène à la sortie de la colonne en fonction de la quantité de benzène alimenté.

Après passage de 7 litres de benzène, la zéolite est saturée à 75% et on arrête l'alimentation. La composition moyenne du benzène passé sur la zéolite Y est comparée à celle du benzène alimenté, comme indiqué dans le tableau suivant :

| | Benzène alimenté | Benzène après passage sur zéolite Y |
|---|---|---|
| Thiophène (ppm) | 4320 | 1916 |
| Dicyclopentadiène (ppm) | 586 | 0 |
| Non-aromatiques (ppm) | 4910 | 3789 |
| Toluène (ppm) | 120 | 111 |
| Benzène (%) | 99,01 | 99,43 |

Un avantage substantiel du procédé suivant l'invention provient du fait que le traitement du benzène impur peut être effectué avec la matière à traiter telle quelle, c'est-à-dire qu'il n'est nullement nécessaire de diluer le benzène impur par un solvant ou par un gaz porteur. Ceci simplifie considérablement les opérations et offre des avantages économiques importants et évite notamment de devoir séparer un solvant ou un gaz porteur du benzène après le traitement par la zéolite.

Un autre avantage du procédé suivant l'invention est que la zéolite utilisée permet d'adsorber non seulement le thiophène et d'autres impuretés sulfurées, mais également une partie des impuretés non sulfurées comme le cyclopentadiène et des hydrocarbures aliphatiques. De ce fait, le raffinage final, par distillation, du benzène traité par la zéolite est fortement facilité.

Suivant l'origine des benzols bruts, les conditions opératoires de l'épuration du benzène impur par la zéolite (en particulier les dimensions de la colonne de zéolite et le débit de benzène au travers de celle-ci) et le degré de pureté désiré pour le benzène épuré, le procédé suivant l'invention permet de simplifier fortement et éventuellement même supprimer cette dernière phase du traitement des benzols bruts. Lorsque, après le traitement du benzène impur par la zéolite, un raffinage final, par distillation, reste nécessaire, celui-ci peut se faire dans une colonne plus petite et de manière plus économique que dans les procédés d'épuration antérieurement connus.

Les essais effectués ont en effet montré que le traitement du benzène impur par le procédé suivant l'invention permet d'obtenir non seulement une élimination pratiquement totale du thiophène, mais également une importante réduction de la teneur en cyclopentadiène et en hydrocarbures aliphatiques.

Il ressort des résultats des essais que le procédé suivant l'invention procure un moyen très efficace et économiquement avantageux pour purifier du benzène, en raison :

- du coût économiquement acceptable et de la facilité d'acquisition des matières utilisées dans le procédé (zéolite Y, sel de cuivre),
- de la facilité de préparation du substrat (par échange d'ions),
- de la grande stabilité du substrat,
- de la simplicité de régénération du substrat (par traitement thermique),
- de la possibilité d'atteindre une élimination pratiquement totale du thiophène et une réduction de la teneur en autres impuretés,
- de l'absence de déchets.

## Revendications

1. Procédé pour l'épuration de benzène impur contenant du thiophène et d'autres impuretés, caractérisé en ce qu'il comprend
- la mise en contact de benzène impur à épurer avec une zéolite d'alumino-silicate cristalline Y qui a subi un échange d'ions avec une solution aqueuse d'un composé de cuivre bivalent, un séchage et un traitement thermique progressif de conditionnement jusqu'à une température comprise entre 200 et 550°C, et
- la régénération de la zéolite chargée de thiophène et d'autres impuretés, comprenant un traitement thermique progressif de régénération jusqu'à une température comprise entre 200 et 550°C pour préparer la zéolite à une nouvelle mise en contact avec du benzène impur à épurer.

2. Procédé suivant la revendication 1, caractérisé en ce que le séchage de la zéolite se fait à l'air pendant au moins 12 heures à environ 50°C.

3. Procédé suivant l'une quelconque des revendications précédentes, caractérisé en ce que le traitement thermique de conditionnement se fait dans une colonne, en faisant passer dans celle-ci un courant d'air sec dont on augmente la température à une allure ne dépassant pas 5°C par minute jusqu'à environ 140°C, en maintenant la température à environ 140°C jusqu'à ce que l'air sortant de la colonne ne contienne plus d'humidité, en augmentant de nouveau la température du courant d'air à une allure ne dépassant pas 10°C par minute jusqu'à une température comprise entre 200 et 550°C, en maintenant le courant d'air à cette température, au moins jusqu'à ce qu'il y ait uniformité de température dans la colonne, et en refroidissant finalement la colonne par passage d'un courant d'air sec non chauffé.

4. Procédé suivant l'une quelconque des revendications précédentes, caractérisé en ce que le traitement thermique de conditionnement se fait jusqu'à une température comprise entre 300 et 400°C.

5. Procédé suivant la revendication 4, caractérisé en ce que le traitement thermique de conditionnement se fait jusqu'à environ 350°C.

6. Procédé suivant l'une quelconque des revendications précédentes, caractérisé en ce que le benzène impur à épurer est mis en contact avec la zéolite à l'état liquide à une température comprise entre 20 et 80°C à pression atmosphérique.

7. Procédé suivant l'une quelconque des revendications 1 à 5, caractérisé en ce que le benzène impur à épurer est mis en contact avec la zéolite à l'état liquide, sous pression, à une température supérieure à 80°C.

8. Procédé suivant l'une quelconque des revendications 1 à 5, caractérisé en ce que le benzène impur à épurer est mis en contact avec la zéolite à l'état de vapeur.

9. Procédé suivant l'une quelconque des revendications précédentes, caractérisé en ce que la mise en contact du benzène impur à épurer avec la zéolite se fait en continu en faisant passer le benzène impur dans une colonne chargée de la dite zéolite.

10. Procédé suivant la revendication 9, caractérisé en ce que pour la régénération de la zéolite, on chasse les liquides et les vapeurs de la colonne avec un courant d'azote dont on augmente la température à une allure ne dépassant par 10°C par minute jusqu'à environ 150°C, on remplace le courant d'azote par un courant d'air sec dont on continue à augmenter la température à une allure ne dépassant pas 10°C par minute jusqu'à une température comprise entre 200 et 550°C, on maintient le courant d'air à cette température, au moins jusqu'à ce qu'il y ait uniformité de température dans la colonne, et on refroidit finalement la colonne jusqu'à la température d'utilisation de la zéolite, par passage d'un courant d'air sec non chauffé.

11. Procédé suivant la revendication 10, caractérisé en ce que le traitement thermique de régénération se fait jusqu'à une température comprise entre 300 et 400°C.

12. Procédé suivant la revendication 11, caractérisé en ce que le traitement thermique de régénération se fait jusqu'à 350°C.

13. Procédé suivant l'une quelconque des revendications 6 et 7, caractérisé en ce que la mise en contact du benzène impur à épurer avec la zéolite se fait en discontinu, en ajoutant la zéolite au benzène impur et en le maintenant pendant un certain temps sous agitation.

## Patentansprüche

1. Verfahren zur Reinigung von Thiophen und andere Verunreinigungen enthaltendem Benzol, dadurch gekennzeichnet, daß das zu reinigende verunreinigte Benzol mit einem kristallinen Aluminosilicat-Zeolith Y, welcher einem Ionenaustausch mit einer wässerigen Lösung einer Cu(II)-verbindung, einer

Trocknung mit einer fortschreitenden thermischen Konditionierungsbehandlung bis zu einer Temperatur zwischen 200 und 550°C unterworfen worden war, in Berührung gebracht wird, der mit Thiophen und anderen Verunreinigungen beladenen Zeolith durch eine fortschreitende thermische Regenerierungsbehandlung bis zu einer Temperatur zwischen 200 und 550°C regeneriert wird, um ein Zeolith für ein neuerliches In-Berührung-bringen mit dem zu reinigenden verunreinigten Benzol zu erhalten.

2. Verfahren nach Anspruch 1, dadurch gekennzeichnet, daß die Trocknung des Zeoliths in Luft während mindestens 12 Stunden bei ungefähr 50°C durchgeführt wird.

3. Verfahren nach einem der vorhergehenden Ansprüche, dadurch gekennzeichnet, daß die thermische Konditionierungsbehandlung in einer Säule erfolgt, indem durch diese ein trockener Luftstrom geführt wird, dessen Temperatur man in einem 5°C pro Minute nicht übersteigenden Ausmaß bis ungefähr 140°C erhöht, die Temperatur von ungefähr 140°C aufrecht erhalten wird bis die aus der Säule herauskommende Luft keine Feuchtigkeit mehr enthält, die Temperatur des Luftstromes in einem 10°C pro Minute nicht übersteigenden Ausmaß erneut bis auf eine Temperatur zwischen 200 und 550°C erhöht wird, der Luftstrom auf dieser Temperatur mindestens so lange gehalten wird, bis in der Säule gleichmäßige Temperatur herrscht und schließlich die Säule durch Durchleiten eines trockenen, nicht erwärmten Luftstromes gekühlt wird.

4. Verfahren nach einem der vorhergehenden Ansprüche, dadurch gekennzeichnet, daß die thermische Konditionierungsbehandlung bei einer Temperatur zwischen 300 und 400°C durchgeführt wird.

5. Verfahren nach Anspruch 4, dadurch gekennzeichnet, daß die thermische Konditionierungsbehandlung bei ungefähr 350°C durchgeführt wird.

6. Verfahren nach einem der vorhergehenden Ansprüche, dadurch gekennzeichnet, daß das zu reinigende verunreinigte Benzol mit dem Zeolith in flüssigem Zustand bei einer Temperatur zwischen 20 und 80°C bei Atmosphärendruck in Berührung gebracht wird.

7. Verfahren nach einem der Ansprüche 1 bis 5, dadurch gekennzeichnet, daß das zu reinigende verunreinigte Benzol mit dem Zeolith in flüssigem Zustand unter Druck bei einer Temperatur über 80°C in Berührung gebracht wird.

8. Verfahren nach einem der Ansprüche 1 bis 5, dadurch gekennzeichnet, daß das zu reinigende verunreinigte Benzol mit dem Zeolith in dampfförmigem Zustand in Berührung gebracht wird.

9. Verfahren nach einem der vorhergehenden Ansprüche, dadurch gekennzeichnet, daß das zu reinigende verunreinigte Benzol mit dem Zeolith kontinuierlich in Berührung gebracht wird, indem das verunreinigte Benzol durch eine mit dem Zeolith beschickte Säule geführt wird.

10. Verfahren nach Anspruch 9, dadurch gekennzeichnet, daß man zur Regenerierung des Zeoliths die Flüssigkeiten und Dämpfe aus der Säule mit einem Stickstoffstrom, dessen Temperatur man in einem 10°C pro Minute nicht übersteigenden Ausmaß bis ungefähr 150°C erhöht, austreibt, den Stickstoffstrom durch einen trockenen Luftstrom, dessen Temperatur man kontinuierlich in einem 10°C pro Minute nicht übersteigenden Ausmaß bis auf eine Temperatur zwischen 200 und 550°C erhöht, ersetzt, den Luftstrom auf diese Temperatur mindestens so lange hält, bis in der Säule eine gleichmäßige Temperatur herrscht, und die Säule auf die Verwendungstemperatur des Zeoliths durch Hindurchleiten eines trockenen, nicht erwärmten Luftstroms abkühlt.

11. Verfahren nach Anspruch 10, dadurch gekennzeichnet, daß die thermische Regenerierungsbehandlung bei einer Temperatur zwischen 300 und 400°C durchgeführt wird.

12. Verfahren nach Anspruch 11, dadurch gekennzeichnet, daß die thermische Regenerierungsbehandlung bis zu 350°C durchgeführt wird.

13. Verfahren nach Anspruch 6 oder 7, dadurch gekennzeichnet, daß das zu reinigende verunreinigte Benzol mit dem Zeolith diskontinuierlich in Berührung gebracht wird, indem der Zeolith dem verunreinigten Benzol hinzugefügt und das Gemisch während einer bestimmten Zeit gerührt wird.

**Claims**

1. A method for the purification of impure benzene containing thiophene and other impurities, characterised in that it comprises the bringing-into-contact of impure benzene, to be purified, with a crystalline alumino-silicate Y zeolite which has undergone an ion exchange with an aqueous solution of a bivalent copper compound, a drying process and a progressive thermal conditioning treatment up to a temperature of between 200 and 550°C, wherein regeneration of the zeolite, laden with thiophene and other impurities, comprises a progressive thermal regeneration treatment up to a temperature of between 200 and 550°C in order to prepare the zeolite for being brought again into contact with impure benzene to be purified.

2. The method according to claim 1, characterised in that drying of the zeolite takes place in air for at least 12 hours at approximately 50°C.

3. The method according to anyone of the preceding claims, characterised in that the thermal conditioning treatment takes place in a column through which a current of dry air is passed whose temperature is increased at a rate not exceeding 5°C per minute up to approximately 140°C, the temperature being maintained at approximately 140°C until the air leaving the column does not contain any more moisture, the temperature of the air current being increased again at a rate not exceeding 10°C per minute up to a level of between 200 and 550°C, the air current then being maintained at this temperature at least until there is uniformity of temperature in the column, the column being finally cooled by the passage of a dry, unheated, air current.

4. The method according to anyone of the preceding claims, characterised in that the thermal conditioning treatment takes place up to a temperature of between 300 and 400°C.

5. The method according to claim 4, characterised in that the thermal conditioning treatment takes place up to approximately 350°C.

6. The method according to anyone of the preceding claims, characterised in that the impure benzene to be purified is brought into contact with the zeolite in liquid state at a temperature of between 20 and 80°C at atmospheric pressure.

7. The method according to anyone of claims 1 to 5, characterised in that the impure benzene to be purified is brought into contact with the zeolite in liquid state under pressure at a temperature higher than 80°C.

8. The method according to anyone of claims 1 to 5, characterised in that the impure benzene to be purified is brought into contact with the zeolite in vaporous state.

9. The method according to anyone of the preceding claims, characterised in that the bringing-into-contact of the impure benzene, to be purified, with the zeolite occurs continuously in that the impure benzene is passed through a column charged with the said zeolite.

10. The method according to claim 9, characterised in that, for regeneration of the zeolite, the liquids and vapours are driven out of the column with a nitrogen current whose temperature is increased at a rate not exceeding 10°C per minute up to approximately 150°C, in that the nitrogen current is replaced by a current of dry air whose temperature continues to be increased at a rate not exceeding 10°C per minute up to a level of between 200 and 550°C, the air current being maintained at this temperature at least until there is uniformity of temperature in the column, and in that finally the column is cooled down to the temperature of employment of the zeolite by the passage of a dry, unheated, air current.

11. The method according to claim 10, characterised in that the thermal regeneration treatment takes place up to a temperature of between 300 and 400°C.

12. The method according to claim 11, characterised in that the thermal regeneration treatment takes place up to 350°C.

13. The method according to anyone of claims 6 and 7, characterised in that the impure benzene, to be purified, is brought into contact with the zeolite in a discontinuous manner, the zeolite being added to the impure benzene and maintained in agitation for a certain time.

%
100
50
B
H
T
100
200
t (minutes)

Fig. 1.

Fig. 2.
%
100
50
B
H
100
200
t (minutes)

Fig. 5
ppms
4,5
4
3,5
3
2,5
2
1,5
1
0,5
0
0
2,6
3,6
4,6
5,8
6,6
7,35
7,8
8,8
10
LITRES DE BENZÈNE

ppm de thiophène
4500
4000
3500
3000
2500
2000
1500
1000
500
0
1000
3000
5000
7000
LITRES DE BENZENE ALIMENTÉ
COLONNE
CONCENTRATION INITIALE

Fig. 4.